# EUROPÄISCHE PATENTSCHRIFT

(11) **EP 1 718 239 B1**
(45) Veröffentlichungstag und Bekanntmachung des Hinweises auf die Patenterteilung: **12.08.2009**
(21) Anmeldenummer: 05707568.1
(22) Anmeldetag: 22.02.2005
(51) Int. Cl.: A61F 2/00

(54) **IMPLANTIERBARE PROTHESE ZUR REPARATUR VON HERNIENDEFEKTEN**
IMPLANTABLE PROSTHESIS FOR REPAIRING HERNIA DEFECTS
PROTHESE IMPLANTABLE SERVANT A REPARER DES DEFAUTS HERNIAIRES

(30) Priorität: 26.02.2004 DE 102004009892
(43) Veröffentlichungstag der Anmeldung: 08.11.2006
(73) Patentinhaber: GfE Medizintechnik GmbH, 90431 Nürnberg (DE)
(72) Erfinder: ZIMMERMANN, Hanngörg, 91327 Gössweinstein (DE); HEINLEIN, Markus, 91327 Gössweinstein (DE)
(74) Vertreter: Hübner, Gerd
(86) Internationale Anmeldenummer: PCT/EP2005/001829
(87) Internationale Veröffentlichungsnummer: WO 2005/082273

(56) Entgegenhaltungen:
- WO-A-01/97713
- FR-A- 2 778 554
- US-A- 3 431 909
- US-A- 5 716 408

## Beschreibung

Die Erfindung betrifft eine implantierbare Prothese zur Reparatur von Herniendefekten oder vergleichbaren Weichgewebedefekten mit einem Grundkörper aus einem netzförmigen, insbesondere gewirkten Lagenmaterial, der zu einem stopfenartigen, in den Herniendefekt positionierbaren Einsatz deformierbar ist.

Derartige Prothesen, die im Fachjargon auch als "Hernien-Stopfen" oder "Hernien-Plug" bezeichnet werden, sind in vielerlei unterschiedlichen Ausführungsformen bekannt. Als Beispiel kann auf die US 5 716 408 A1, worauf der Oberbegriff des Anspruchs 1 basert, verwiesen werden, die einen Hernien-Plug bestehend aus kegelförmigen, ineinandersitzenden Stopfenelementen offenbart. Die Stopfenelemente sind jeweils durch Plissieren mit einer konischen, in Falten gelegten Wandung versehen. Insoweit ist der dort gezeigte Hernien-Plug in seiner Konstruktion und Herstellung besonders aufwändig, da jedes einzelne Element konisch plissiert, die einzelnen Elemente ineinander gesteckt und anschließend aneinander fixiert werden müssen.

Davon ausgehend liegt der Erfindung die Aufgabe zugrunde, eine implantierbare Prothese zur Reparatur von Herniendefekten so auszugestalten, dass sie ohne signifikante Einbußen bei ihren therapeutischen Wirkungen weitaus einfacher aufgebaut und mit entsprechend geringem Herstellungsaufwand produzierbar, dabei während der Implantation jedoch bequem und sicher handhabbar ist.

Diese Aufgabe wird durch die im Kennzeichnungsteil des Anspruches 1 angegebenen Merkmale gelöst. Demnach ist der Grundkörper der Prothese aus einem vorzugsweise runden bis ovalen Zuschnitt des Lagenmaterials gebildet, das in parallel zueinander verlaufende Zick-Zack-Falten gelegt ist. Insoweit ist bereits gegenüber dem Stand der Technik der Vorteil zu nennen, dass lediglich eine Materiallage für die Herstellung des Grundkörpers notwendig ist.

Die ferner erfindungsgemäß vorhandenen Zick-Zack-Falten sind nur etwa mittig bezogen auf die gewählte Erstreckungsrichtung durch eine die Faltenlagen durchgreifende Fixierung derart festgelegt, dass der Grundkörper in seiner undeformierten Ruheposition in Draufsicht etwas sanduhrförmig ausgebildet ist. Die Taille dieser Konfiguration entsteht durch die Fixierung der Faltenlagen zueinander, von wo aus die Zick-Zack-Falten zum Rand des Zuschnitts hin mehr oder weniger stark auslaufen.

Von der Handhabung her ist diese Prothesenkonfiguration besonders einfach, die Prothese wird im Bereich der mittigen Fixierung mit zwei Fingern ergriffen und unter Aufbiegen der verbleibenden Bereiche stopfenartig deformiert. Durch die "Raffung" der Falten im zentralen Bereich der Prothese werden ferner hohe Rückstellkräfte gegen die vorstehend erwähnte Deformation generiert, was einem wirksamen Verspreizen der Prothese im Herniendefekt zugute kommen.

Durch die mittige Fixierung der Zick-Zack-Falten kann sich ferner das Lagenmaterial mit zunehmenden Abstand von der Fixierung wieder breiter legen, sodass die sanduhrförmige Konfiguration des Grundkörpers in seiner undeformierten Ruheposition erzeugt wird. Der "Zwickel" im Bereich der Fixierung ist aufgrund seiner eingeschnürten Form besonders gut mit Daumen und Zeigefinger für das Einsetzen in den Herniendefekt zu ergreifen, sodass die erfindungsgemäße Prothese auch handhabungstechnische Vorteile für den Chirurgen in der Anwendung bietet.

Eine besonders wirkungsvolle und zuverlässige, dabei einfach anzulegende Fixierung der Zick-Zack-Falten ist laut den bevorzugten Ausführungsformen gemäß den Ansprüchen 2 bis 4 durch die Verwendung eines Fixierfadens gegeben. Vorzugsweise erstreckt sich die vom Fixierfaden gebildete Fixiernaht quer zur Erstreckungsrichtung der Falten und quer zur Haupterstreckungsebene der undeformierten Prothese. Der Fixierfaden besteht dabei bevorzugtermaßen aus dem gleichen Kunststoffmaterial - vorzugsweise Polypropylen - wie der Lagenmaterialfaden. Dann besteht nämlich die gesamte Prothese aus einem völlig einheitlichen Grundmaterial, was eine erhebliche zulassungsrechtliche Vereinfachung mit sich bringt. Ferner werden bei dieser Ausführungsform mit Fixiernaht die erfindungsgemäßen Prothesen nur durch Fertigungsgänge der klassischen Textilkonfektion, nämlich Zuschneiden, Faltenlegen und Nähen, hergestellt. Es werden komplexere Fertigungsgänge, wie Plissieren, Spritzen oder Gießen, Vorformung von flächigen Zuschnitten in eine konische Grundstruktur und dergleichen, vermieden. Die Prothese ist drapierfähig, weich und auch leicht an Herniennetze annähbar.

In einer alternativen Ausführungsform können der im Wesentlichen zweidimensionale Grundkörper der Prothese zu einer dreidimensional deformierten, stopfenartigen Konfiguration umgeformt werden, indem die benachbarten Seitenkantenzonen beiderseits der Einschnürung vorzugsweise wiederum durch eine Naht aus dem gleichen Fadenmaterial wie der Lagenmaterialfaden verbunden werden. Insoweit bleibt also die "Sortenreinheit" der für die Prothese verwendeten Materialien erhalten.

Gemäß einer weiteren bevorzugten Ausführungsform ist die implantierbare Prothese mit einer durchgehenden körperverträglichen Beschichtung in Form einer Oberflächenmetallisierung, vorzugsweise einer Titan-haltigen Beschichtung, versehen. Damit ist der erfindungsgemäße Hernien-Plug besonders verträglich. Ein weiterer Vorteil der Oberflächenmetallisierung liegt in der damit verbundenen Hydrophilierung des Netzmaterials des Plugs, wodurch sich dieser quasi an die Wände des zu stützenden Herniendefekts saugt. Wegen der mechanischen Verspreizung wird damit die Fixierung des Hernien-Plugs im Defekt weiter verbessert.

Die Konfektionierung des Hernien-Plugs aus einem einlagigen Netzmaterial hat schließlich im Zusammenhang mit der Oberflächenmetallisierung den Vorteil, dass trotz der Faltenlegung die gesamte Netzoberfläche relativ gut zugänglich bleibt, sodass der Metallisierungsprozess etwa mithilfe eines PACVD-Verfahrens, wie es aus der DE 199 45 299 A bekannt ist, flächendeckend durchführbar ist und zu einer geschlossenen Metallisierungsschicht auf dem Kunststoff-Netzmaterial führt. Dies kommt der Gewebeverträglichkeit des Hernien-Plugs weiter zugute.

Schließlich ist gemäß einer weiteren bevorzugten Ausführungsform vorgesehen, das Lagenmaterial für den Grundkörper mithilfe eines Schneidlasers zuzuschneiden. Da dies auf der Basis eines thermischen Schmelzprozesses erfolgt, hat dies den Vorteil, dass an den Schnittkanten keine "Ausfransungen" mit sich lösenden Faserpartikeln, sondern ein sauber abgeschmolzener Randkantenbereich erzielt werden.

Zur Herstellung eines in sich stabileren und stärkeren Hernien-Plugs kann gemäß einer weiteren bevorzugten Ausführungsform vorgesehen sein, zwei in Zick-Zack-Falten gelegte und so fixierte Grundkörper kreuzweise aufeinander zu legen und miteinander zu verbinden, so dass die Grundkonfiguration der Prothese im wesentlichen kleeblattförmig ist.

Ferner kann durch die Verwendung eines in seiner Grundform ovalen, seitlich mit Einschnürungen versehenen Zuschnittes in vorteilhafter Weise beim Raffen der zentralen Falten und deren Vernähung ein übermäßiges Aufbauschen dort im Zentralbereich der Prothese verhindert werden.

Weitere Merkmale, Einzelheiten und Vorteile des Erfindungsgegenstandes sind der nachfolgenden Beschreibung entnehmbar, in der Ausführungsbeispiele des Erfindungsgegenstandes anhand der beigefügten Darstellungen näher erläutert werden.

### Es zeigen:

- Fig.: 1 eine Draufsicht auf einen Hernien-Plug,
- Fig. 2: einen schematischen Querschnitt entlang der Schnittlinie II-II nach Fig. 1,
- Fig. 3: einen schematischen Querschnitt entlang der Schnittlinie III-III nach Fig. 1,
- Fig. 4: eine perspektivische Darstellung eines Hernien-Plugs in einer zweiten Ausführungsform.

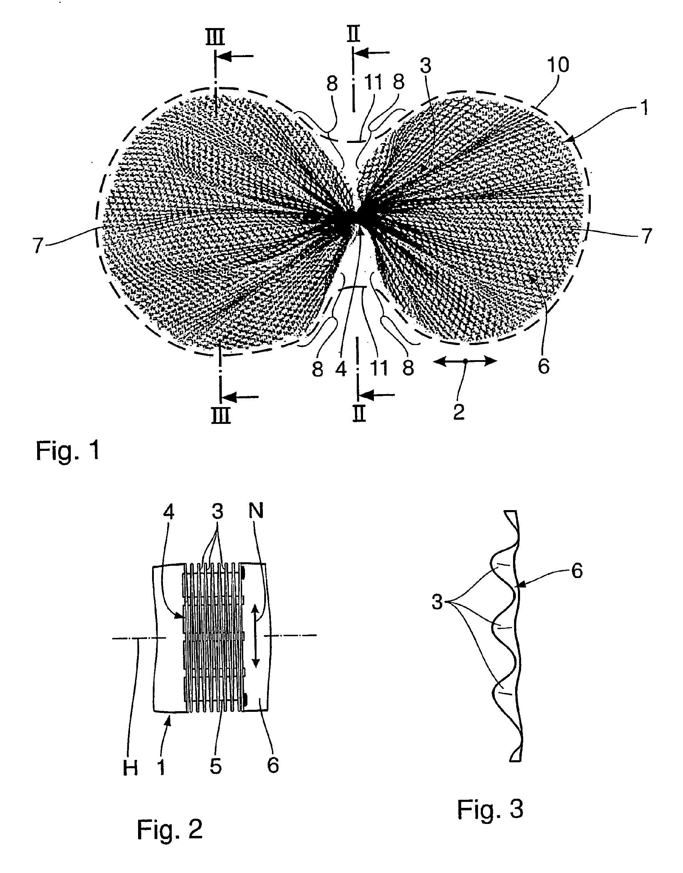

Wie aus Fig. 1 deutlich wird, besteht der gezeigte Hernien-Plug aus einem netzartigen Lagenmaterial 1, das beispielsweise aus einem Polypropylen-Monofilament mit einer Fadenstärke von 100 dtex in Atlas-Legung kettengewirkt ist. Das Flächengewicht dieses Lagenmaterials 1 beträgt ca. 60 - 65 g/m². Daraus wird - wie in Figur 1 durch eine strichlierte Linie dargestellt ist - ein in seiner Grundform ovaler, mit seitlichen Einschnürungen 11 im Mittenbereich sanduhrförmig gestalteter Zuschnitt 10 mittels Laserschneiden auf einem entsprechenden Bahnmaterial herausgeschnitten. Dieser Zuschnitt wird in zur langen Achse der ovalen Grundform parallelen Längserstreckungsrichtung 2 in Zick-Zack-Falten 3 gelegt, wie dies in Fig. 2 schematisch dargestellt ist. Diese Falten 3 bauschen beim Raffen aufgrund der Einschnürungen 11 im Zuschnitt 10 weniger stark auf wie bei einem vollständig ovalen Zuschnitt. Anschließend wird mittig bezogen auf die Längserstreckungsrichtung 2 zur Fixierung der Zick-Zack-Falten 3 eine die Faltenlagen durchgreifende Fixiernaht 4 mithilfe eines monofilen Fadens 5 gelegt. Dieser Faden 5 ist identisch mit dem Fadenmaterial, das zu dem Lagenmaterial 1 verarbeitet wurde, besteht also aus dem gleichen Polypropylen-Granulat und weist eine identische Fadenstärke von 100 dtex auf. Die Nahtrichtung N verläuft quer zur Längser-streckung 2 (Fig. 1) und zur Hauptebene H des Grundkörpers 6 (Fig. 2).
Der wie vorstehend konfektionierte Grundkörper 6 ist - wie aus Fig. 1 deutlich wird - in seiner undeformierten Ruheposition in Draufsicht etwa sanduhrförmig ausgebildet. Die mittig fixierten Zick-Zack-Falten 3 laufen zu den Längsenden 7 des Grundkörpers 6 hin weich in eine mehr oder weniger zweidimensionale Konfiguration aus (siehe Fig. 3).

Der Grundkörper 6 wird nach der Konfektionierung mittels eines aus dem Stand der Technik bekannten PACVD-Prozesses mit einer die gesamte Oberfläche bedeckenden, durchgehenden Titanisierung versehen, deren Spezifikation in der Beschreibungseinleitung bereits näher angegeben wurde.

Im klinischen Einsatz zur Reparatur eines Herniendefektes wird der Grundkörper 6 im Bereich des durch die Fixiernaht 4 gebildeten Zwickels ergriffen und in die Hernienöffnung eingeschoben. Dabei deformiert sich der Grundkörper zu einem stopfenartigen Einsatz und verspreizt sich aufgrund der insbesondere den Falten innewohnenden Rückstellkräfte in der Öffnung.

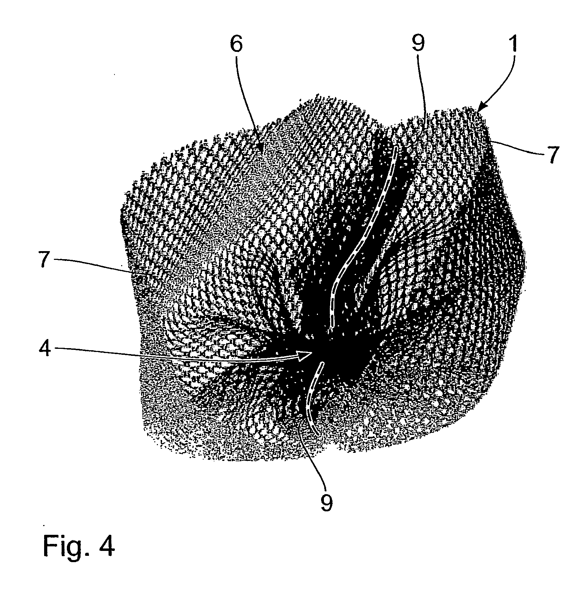

Die in Fig. 4 dargestellte Variante des Hernien-Plugs ist direkt aus der Ausführungsform gemäß Fig. 1 bis 3 entstanden, indem die in Fig. 1 mit dem Bezugszeichen 8 versehenen, benachbarten Seitenkantenzonen durch Deformation des Grundkörpers 6 in Überlappung gebracht und mit einer Naht 9 (strichliert angedeutet in Fig. 4) miteinander verbunden werden. Damit ist der Grundkörper bereits in der stopfenartigen Konfiguration fixiert, in der er zur Reparatur eines Herniendefekts eingesetzt wird. Das Fadenmaterial der Naht 9 ist identisch mit dem für die Fixiernaht 4 im Bereich der Zick-Zack-Falten 3 verwendeten, wodurch diese Variante ebenfalls aus einem einheitlichen Material hergestellt ist.

Auch die in Fig. 4 dargestellte Darstellungsform des Hernien-Plugs wird nach der Formgebung in der dargestellten Weise mit einer Titan-haltigen Beschichtung einer Dicke von < 2 µm, vorzugsweise von 5 bis 700 nm versehen. Praktische Werte der Beschichtungsdicke liegen bei 20 bis 30 nm.

## Patentansprüche

1. Implantierbare Prothese zur Reparatur von Herniendefekten mit einem Grundkörper (6) aus einem netzförmigen, insbesondere gewirkten Lagenmaterial (1), der zu einem stopfenartigen, in den Herniendefekt positionierbaren Einsatz deformierbar ist,
wobei der Grundkörper (6) aus einem vorzugsweise runden bis ovalen Zuschnitt (10) des Lagenmaterials (1) gebildet ist, das in Zick-Zack-Falten (3) gelegt ist, **dadurch gekennzeichnet, dass** die Zick-Zack-Falten parallel zu einer Erstreckungsrichtung (2) verlaufen,
und die Zick-Zack-Falten (3) nur etwa mittig bezogen auf die gewählte Erstreckungsrichtung (2) durch eine die Faltenlagen durchgreifende Fixierung (4) derart festgelegt sind, dass
- der Grundkörper (6) in seiner undeformierten Ruheposition in Draufsicht etwa sanduhrförmig ausgebildet ist.

2. Prothese nach Anspruch 1, **dadurch gekennzeichnet, dass** die Fixierung ein durch die Faltenlagen durchgenähter Fixierfaden (5) ist.

3. Prothese nach Anspruch 2, **dadurch gekennzeichnet, dass** die vom Fixierfaden (5) gebildete Fixiernaht (4) quer zur Erstreckungsrichtung (2) der Falten und quer zur Haupterstreckungsebene (H) der undeformierten Prothese verläuft.

4. Prothese nach Anspruch 2 oder 3, **dadurch gekennzeichnet, dass** der Lagenmaterialfaden und der Fixierfaden (5) aus dem gleichen Kunststoffmaterial, vorzugsweise Polypropylen, bestehen und die gleiche Fadenstärke, vorzugsweise 100 dtex, aufweisen.

5. Prothese nach einem der vorgenannten Ansprüche, **dadurch gekennzeichnet, dass** der Grundkörper (6) durch eine Verbindung der benachbarten Seitenkantenzonen (8) in seiner deformierten stopfenartigen Konfiguration fixiert ist.

6. Prothese nach Anspruch 5, **dadurch gekennzeichnet, dass** die Verbindung der benachbarten Seitenkantenzonen (8) durch eine Naht (9) vorzugsweise aus dem gleichen Fadenmaterial wie der Lagenmaterialfaden hergestellt ist.

7. Prothese nach einem der vorgenannten Ansprüche, **gekennzeichnet durch** eine metallhaltige, durchgehende, körperverträgliche Beschichtung.

8. Prothese nach Anspruch 7, **dadurch gekennzeichnet, dass** die Beschichtung eine Titan-haltige Beschichtung mit einer Dicke von kleiner 2 µm, vorzugsweise von 5 bis 700 nm ist.

9. Prothese nach einem der vorgenannten Ansprüche, **dadurch gekennzeichnet, dass** das Lagenmaterial (1) für den Grundkörper (6) Laserzugeschnitten ist.

10. Prothese nach einem der vorgenannten Ansprüche, **dadurch gekennzeichnet, dass** zwei Grundkörper (6) kreuzweise aufeinander gelegt und miteinander verbunden sind.

11. Prothese nach einem der vorgenannten Ansprüche, **dadurch gekennzeichnet, dass** der noch nicht in Falten gelegte Zuschnitt (10) des Grundkörpers (6) eine runde bis ovale Außenkontur mit einer sanduhrförmigen Einschnürung (11) im Mittenbereich aufweist.

## Claims

1. An implantable prosthesis for repairing hernia defects, comprising a basic structure (6) of meshed, in particular knitted, layer material (1) which is deformable into a plug-type insert capable of being positioned in the hernia defect, wherein the basic structure (6) is a blank (10) of the layer material (1) of a shape preferably ranging from round to oval, which is placed in concertina pleats (3), **characterized in that** the concertina pleats (3) are parallel to a direction of extension (2); and **in that** the concertina pleats (3) are fixed only approximately centrally as related to the selected direction of extension (2) by a fixing arrangement (4) that passes through the pleats in such a way that
- the basic structure (6), in its undeformed position of rest, is approximately hourglass-shaped in a plan view.

2. A prosthesis according to claim 1, **characterized in that** the fixing arrangement is a fixing thread (5) that is stitched through the pleated layers.

3. A prosthesis according to claim 2, **characterized in that** the fixing seam (4) that is formed by the fixing thread (5) runs crosswise of the direction of extension (2) of the pleats and crosswise of the principal plane (H) of extension of the undeformed prosthesis.

4. A prosthesis according to claim 2 or 3, **characterized in that** the thread of layer material and the fixing thread (5) consist of the same plastic material, preferably polypropylene, and have the same thread thickness, preferably 100 dtex.

5. A prosthesis according to one of the preceding claims, **characterized in that** the basic structure (6) is fixed in its deformed, plug-type configuration by connection of adjoining lateral-edge areas (8).

6. A prosthesis according to claim 5, **characterized in that** the connection of the adjoining lateral-edge areas (8) is produced by a seam (9), preferably of the same thread material as the layer-material thread.

7. A prosthesis according to one of the preceding claims, **characterized by** a metal-containing, continuous, biocompatible coating.

8. A prosthesis according to claim 7, **characterized in that** the coating is a titanium-containing coating of a thickness of less than 2 µm, preferably 5 to 700 nm.

9. A prosthesis according to one of the preceding claims, **characterized in that** the layer material (1) for the basic structure (6) is laser-beam cut to size.

10. A prosthesis according to one of the preceding claims, **characterized in that** two basic structures (6) are placed crosswise one on top of the other and joined to each other.

11. A prosthesis according to one of the preceding claims, **characterized in that** the blank (10), not yet pleated, of the basic structure (6) has an outer contour ranging from round to oval with a constriction (11) of hourglass shape in the central area.

## Revendications

1. Prothèse implantable, destinée à la réparation des lésions herniaires, comportant un corps de base (6), réalisé dans un matériau stratifié (1) en forme de filet, en particulier de type tricot, qui peut être déformé pour former un insert en forme de bouchon, destiné à être positionné dans la lésion herniaire, le corps de base (6) étant formé par une découpe (10), de préférence ronde à ovale, du matériau stratifié (1), qui est plié en formant des plis en accordéon (3),
**caractérisée en ce que**
- les plis en accordéon (3) sont orientés parallèlement à une direction en longueur (2),
- et les plis en accordéon (3) sont fixés, seulement sensiblement au milieu par rapport à la direction en longueur (2) choisie, par une fixation (4) traversant les couches de plis, de telle sorte que
- le corps de base (6), dans sa position de repos non déformée, est réalisé sensiblement avec une forme en diabolo sur une vue en élévation.

2. Prothèse selon la revendication 1, **caractérisée en ce que** la fixation est un fil de fixation (5) cousu en passant à travers les couches de plis.

3. Prothèse selon la revendication 2, **caractérisée en ce que** la couture de fixation (4) formée par le fil de fixation (5), est orientée transversalement à la direction en longueur (2) des fils et transversalement au plan en longueur principal (H) de la prothèse non déformée.

4. Prothèse selon la revendication 2 ou 3, **caractérisée en ce que** le fil du matériau stratifié et le fil de fixation (5) sont réalisés dans le même matériau plastique, de préférence du polypropylène, et ont la même grosseur, de préférence 100 dtex.

5. Prothèse selon l'une quelconque des revendications précédentes, **caractérisée en ce que** le corps de base (6) est fixé dans sa configuration déformée en forme de bouchon par un assemblage des zones de bordure latérale (8) adjacentes.

6. Prothèse selon la revendication 5, **caractérisée en ce que** l'assemblage des zones de bordure latérale (8) adjacentes est formé par une couture (9), de préférence avec un fil du même matériau que le fil du matériau stratifié.

7. Prothèse selon l'une quelconque des revendications précédentes, **caractérisée par** un revêtement biocompatible, continu, contenant du métal.

8. Prothèse selon la revendication 7, **caractérisée en ce que** le revêtement est un revêtement contenant du titane et ayant une épaisseur inférieure à 2 µm, de préférence de 5 à 700 nm.

9. Prothèse selon l'une quelconque des revendications précédentes, **caractérisée en ce que** le matériau stratifié (1) pour le corps de base (6) est découpé au laser.

10. Prothèse selon l'une quelconque des revendications précédentes, **caractérisée en ce que** deux corps de base (6) sont posés en croix l'un sur l'autre et sont assemblés l'un à l'autre.

11. Prothèse selon l'une quelconque des revendications précédentes, **caractérisée en ce que** la découpe (10) du corps de base (6), non encore posée en plis, possède un contour extérieur rond à ovale avec un étranglement (11) en forme de diabolo dans la zone centrale.
